# EUROPEAN PATENT APPLICATION

(11) **EP 4 302 807 A1**
(43) Date of publication of application: **10.01.2024**
(21) Application number: 22763201.5
(22) Date of filing: 28.02.2022
(51) Int. Cl.: A61M 5/30, A61M 5/42

(54) **AID TO BE APPLIED TO NEEDLELESS SYRINGE, NEEDLELESS SYRINGE PROVIDED WITH AID, AND INTRADERMAL INJECTION METHOD**

(30) Priority: 01.03.2021 JP 2021032022
(71) Applicant: Daicel Corporation, Osaka 530-0011 (JP)
(72) Inventor: YAMASHITA, Kunihiko, Tokyo 108-8230 (JP); MIKI, Katsuya, Tokyo 108-8230 (JP)
(74) Representative: Grünecker Patent- und Rechtsanwälte PartG mbB
(86) International application number: PCT/JP2022/008323
(87) International publication number: WO 2022/186145

(57) **Abstract**

An aid of a jet injector includes a first pinching piece and a second pinching piece that face each other, and pinches a skin in a state of holding the skin with pinching surfaces facing each other and thus forms a fold shape part where the skin is raised in a fold shape; and a reception portion that is provided in the first pinching piece, exposes a fold top part vicinity region, which is a region in a vicinity of a fold top part, in the fold shape part, and can receive the nozzle portion of the jet injector.

## Description

### Technical Field

The present invention relates to an aid to be applied to a jet injector, a jet injector with an aid, and an intradermal injection method.

### Background Art

As a device for intradermally injecting an injection objective substance such as a medicinal solution into a living body or the like, a jet injector without an injection needle is exemplified. In general, for a jet injector, a structure is employed in which an injection objective substance pressurized by a drive source such as a compressed gas or a spring is injected toward a target region, and the injection objective substance is injected intradermally using kinetic energy of the injection objective substance.

In this connection, for example, Patent Document 1 discloses a technique in which a cylindrical skirt is formed on a distal end side of a jet injector and an injection tube is disposed in a compartment defined by the skirt. In the jet injector, air in the compartment is sucked by a suction pump in a state where an open end of the skirt is pressed against a patient's skin. Due to this, the patient's skin is sucked into the compartment, and the skin near the distal end of the injection tube is maintained in a stable state.

### Citation List

### Patent Documents

Patent Document 1: US 6,406,456
Patent Document 2: WO 2012/036264
Patent Document 3: WO 2019/004322
Patent Document 4: WO 2009/111794
Patent Document 5: JP 2007-61577 A
Patent Document 6: JP 2007-267838 A

### Summary of Invention

### Technical Problem

In general, the skin of a mammal is composed of three layers of an epidermis, which is an outermost layer, a dermis, which is a layer beneath the epidermis, and a subcutaneous tissue, which is a tissue beneath the dermis. It is known that by administering a specific injection objective substance such as a vaccine or a gene therapy agent to the epidermis or the dermis, a response to the injection objective substance is more effectively achieved as compared with a case of administering the injection objective substance to a subcutaneous tissue or a muscle layer. Therefore, also for a jet injector, it may be preferable to intensively inject an injection objective substance into the epidermis or the dermis. Hitherto, it has been difficult to perform administration to the epidermis and the dermis with a jet injector, and precise output control or the like according to an animal species has been required.

The technique according to the present disclosure has been made in view of the above circumstances, and an object thereof is to provide a technique that allows intensive and reliable injection of an injection objective substance into the epidermis or the dermis in intradermal injection to a mammal with a jet injector.

### Solution to Problem

To solve the above problems, the technique according to the present disclosure employs the following configuration. That is, the technique according to the present disclosure is an aid to be applied to a jet injector that intradermally injects an injection objective substance by injecting the injection objective substance from an injection port formed in a nozzle portion, the aid including a first pinching piece and a second pinching piece that face each other, and pinch a skin in a state of holding the skin with pinching surfaces facing each other and thus form a fold shape part where the skin is raised in a fold shape; and a reception portion that is provided in the first pinching piece, exposes a fold top part vicinity region, which is a region in a vicinity of a fold top part, in the fold shape part, and can receive the nozzle portion.

When the aid according to the present disclosure is used and the injection objective substance is injected from the injection port formed in the nozzle portion in a state where the nozzle portion of the jet injector is received in the reception portion of the aid, the injection objective substance is injected to the fold top part vicinity region. Here, since the fold shape part is formed in a state where the skin is held, the fold top part vicinity region includes the epidermis and the dermis and does not include the subcutaneous tissue. As a result, the aid according to the present disclosure allows intensive and reliable injection of the injection objective substance into the epidermis or the dermis.

For the aid according to the present disclosure, the fold top part vicinity region may be a region positioned in a range within 3 mm from the fold top part to a fold root part side in the fold shape part.

The aid according to the present disclosure may further include a positioning portion that is provided on the pinching surface of at least one of the first pinching piece or the second pinching piece and positions the fold top part by abutting on the fold top part of the fold shape part.

In the aid according to the present disclosure, the reception portion may position the injection port with respect to the fold top part vicinity region by being engaged with the nozzle portion of the jet injector.

In the aid according to the present disclosure, the reception portion may position the injection port by being engaged with the nozzle portion of the jet injector and the injection port may be positioned within 3 mm from the fold top part to the fold root part side in the fold shape part.

Furthermore, in the aid according to the present disclosure, the reception portion may position the injection port by being engaged with the nozzle portion of the jet injector, and the injection port may be positioned within 1.5 mm from the fold top part to the fold root part side in the fold shape part.

In the aid according to the present disclosure, the pinching surface of the second pinching piece may be formed as a flat surface.

In the aid according to the present disclosure, the pinching surface of at least one of the first pinching piece or the second pinching piece may be provided with a non-slip portion for suppressing slippage of the fold shape part.

The aid according to the present disclosure may include a first extension part extending from one end of the first pinching piece and provided with a first grasping part at least in a part of the first extension part, a second extension part extending from one end of the second pinching piece and provided with a second grasping part at least in a part of the second extension part, and a coupling portion coupling the first extension part and the second extension part such that the first pinching piece and the second pinching piece open and close by an operation of the first grasping part and the second grasping part.

In the aid according to the present disclosure, the first extension part may extend such that the nozzle portion does not interfere with the first extension part when the nozzle portion is received in the reception portion.

In the aid according to the present disclosure, the first extension part may extend from the first pinching piece and the first pinching piece and the first extension part may form a straight line shape, and the second extension part may extend from the second pinching piece and the second pinching piece and the second extension part may form a V shape.

The technique according to the present disclosure can also be identified as a jet injector with an aid. That is, the technique according to the present disclosure may be a jet injector with an aid that includes a jet injector that intradermally injects an injection objective substance by injecting the injection objective substance from an injection port formed in a nozzle portion, and an aid coupled to the jet injector, in which the aid includes a first pinching piece and a second pinching piece that face each other, and pinch a skin in a state of holding the skin with pinching surfaces facing each other and thus form a fold shape part where the skin is raised in a fold shape, and a reception portion that is provided in the first pinching piece and receives the nozzle portion such that the injection port faces a fold top part vicinity region, which is a region in a vicinity of a fold top part, in the fold shape part.

The technique according to the present disclosure can also be identified as an injection method. That is, the technique according to the present disclosure may be an injection method for intradermally injecting an injection objective substance to a mammal (excluding a human) by injecting the injection objective substance from an injection port formed in a nozzle portion of a jet injector, the injection method including preparing the jet injector, and an aid including a first pinching piece and a second pinching piece facing each other and a reception portion that is provided in the first pinching piece and can receive the nozzle portion; pinching a skin in a state of holding the skin with pinching surfaces facing each other of the first pinching piece and the second pinching piece and thus forming a fold shape part in which the skin is raised in a fold shape, and exposing, through the reception portion, a fold top part vicinity region, which is a region in a vicinity of a fold top part, in the fold shape part; and receiving the nozzle portion of the jet injector in the reception portion in a state where the skin is pinched, and injecting the injection objective substance from the injection port to the fold top part vicinity region.

In the injection method according to the present disclosure, in injecting the injection objective substance from the injection port, the injection objective substance may be injected at a position within 3 mm from the fold top part to a fold root part side in the fold shape part.

In the injection method according to the present disclosure, in injecting the injection objective substance from the injection port, the injection objective substance may be injected at a position within 1.5 mm from the fold top part to a fold root part side in the fold shape part.

In the injection method according to the present disclosure, the injection objective substance may contain a gene.

### Advantageous Effects of Invention

The technique according to the present disclosure allows intensive and reliable injection of an injection objective substance into the epidermis or the dermis in intradermal injection to a mammal with a jet injector.

### Brief Description of Drawings

FIG. 1 is an overall view of an aid according to a first embodiment.
FIG. 2 is an overall view of a jet injector to which the aid according to the first embodiment is applied.
FIG. 3 is a cross-sectional view of the jet injector.
FIG. 4 is a top view of the aid in a closed state.
FIG. 5 is a side view of the aid in a closed state.
FIG. 6 is a side view of the aid in an open state.
FIG. 7 is a flowchart showing a procedure of an injection method using the aid according to the first embodiment.
FIG. 8 is a cross-sectional view illustrating a state in which a skin is pinched by the aid according to the first embodiment.
FIG. 9 is an enlarged view of FIG. 8.
FIG. 10 is a top view illustrating a state in which a skin is pinched by the aid according to the first embodiment.
FIG. 11 is a cross-sectional view illustrating a state in which a nozzle of the injector according to the first embodiment is received in a reception portion of the aid.
FIG. 12 is a top view illustrating a state in which a nozzle portion of the jet injector is received in the reception portion of the aid according to the first embodiment.
FIG. 13 is a graph showing relative light units of Examples 1 to 4 and Comparative Example.
FIG. 14 is a cross-sectional view of an aid according to a first modified example of the first embodiment.
FIG. 15 is a top view illustrating a state in which the nozzle portion of the jet injector is received in a reception portion of an aid according to a second modified example of the first embodiment.
FIG. 16 is a top view illustrating a state in which the nozzle portion of the jet injector according to a modified example is received in a reception portion of an aid according to a third modified example of the first embodiment.
FIG. 17 is an overall view of an aid according to a second embodiment.
FIG. 18 is a cross-sectional view of a jet injector with an aid according to a third embodiment.

Embodiments of the present disclosure will be described below with reference to the drawings. Note that each of the configurations, combinations thereof, and the like in each embodiment is an example, and additions, omissions, substitutions, and other changes of the configuration may be made as appropriate without departing from the spirit of the present invention. The present disclosure is not limited by the embodiments, but only limited by the claims.

### <First Embodiment>

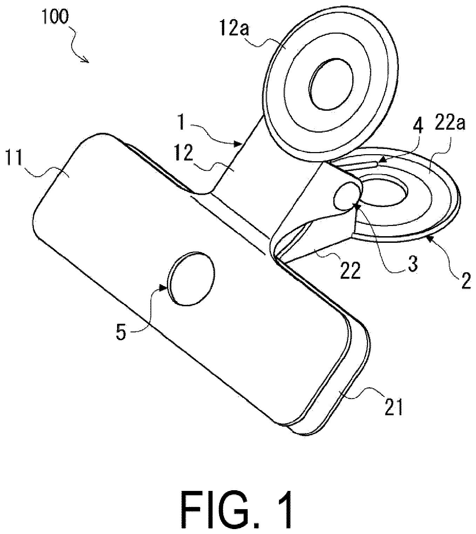

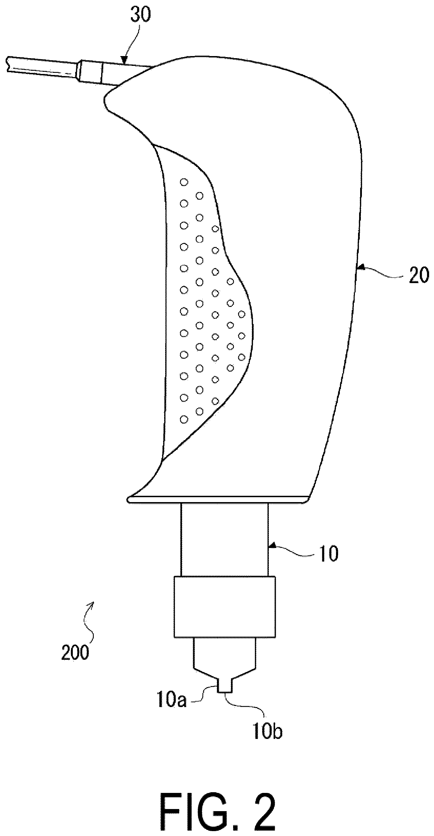

FIG. 1 is an overall view of an aid 100 according to the first embodiment. FIG. 2 is an overall view of a jet injector (hereinafter, simply referred to as an "injector") 200 to which the aid 100 according to the first embodiment is applied. The injector 200 illustrated in FIG. 2 is a jet injector that injects an injection objective substance into the skin (hereinafter, simply referred to as "intradermal") of a mammal from an injection port 10b formed in a nozzle portion denoted by a reference sign 10a, using combustion energy of an explosive. That is, the injector 200 is a device that performs injection by intradermally injecting the injection objective substance without use of an injection needle. The aid 100 according to the present embodiment aids intradermal injection of the injection objective substance to a mammal with the injector 200, thereby enabling injection of the injection objective substance into the epidermis or the dermis. In the present specification, the term "mammal" is not particularly limited, but examples thereof include humans and mammals other than humans. Examples of mammals other than humans include mice, rats, guinea pigs, hamsters, cows, goats, sheep, pigs, monkeys, dogs, and cats.

### [Injector 200]

First, the injector 200 will be described. In the present specification, "distal end side" and "proximal end side" are used as terms indicating a relative positional relationship in a long direction of the injector 200. The "distal end side" refers to the injection port 10b side in the long direction of the injector 200, and the "proximal end side" refers to the side opposite to the injection port 10b side in the long direction of the injector 200 (side of an initiator 101b described later).

In the present specification, it does not matter whether the intradermal injection with the injector 200 is performed in an in vivo environment or in vitro environment. The injection objective substance may have any specific content or form, the component that should be administered intradermally may be dissolved or need not be dissolved, and various forms such as a liquid, a gel, and a suspension can be employed.

The injection objective substance to be injected intradermally with the injector 200 in the present embodiment is formed by containing, in a liquid medium, a predetermined substance that exerts intradermally expected efficacy and function. Examples of the predetermined substance contained in the injection objective substance include a bio-based substance that can be injected into a living body and a substance that exhibits a desired physiological activity. Examples of the bio-based substance include DNA, RNA, nucleic acid, antibody, cell, and the like. Examples of the substance that exhibits a physiological activity include a substance including a low molecule, a middle molecule, a polymer, protein, peptide, or the like, an inorganic substance such as a vaccine, a metal particle for hyperthermia or radiotherapy, and a substance including a carrier and having various pharmacological or therapeutic effects. The liquid that is a medium of the injection objective substance is any substance suitable for intradermally administering these predetermined substances, and may be aqueous or oily. As long as the predetermined substance can be injected with the injector 200, the viscosity of the liquid that is the medium is not particularly limited.

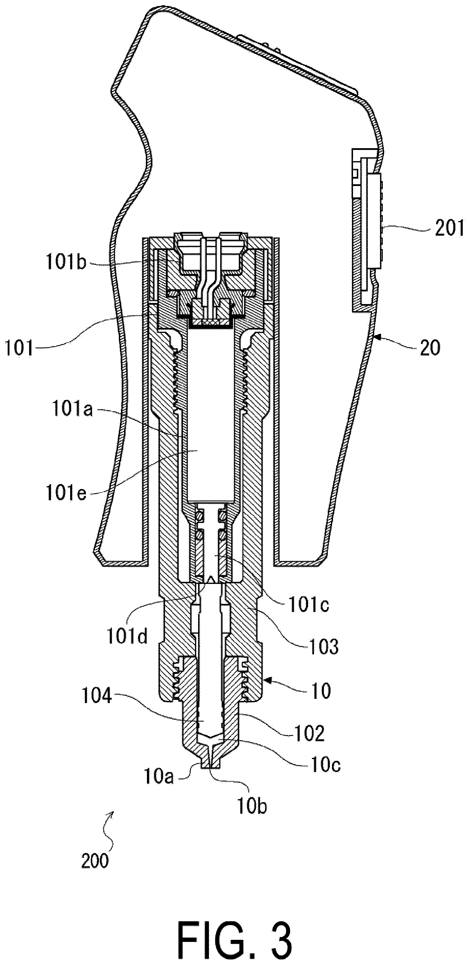

As illustrated in FIG. 2, the injector 200 includes an injector assembly 10, a housing 20 accommodating the injector assembly 10, and a power cable 30 for supplying a drive current to the injector assembly 10 in the housing 20. The injector 200 injects the injection objective substance from the injection port 10b formed in the injector assembly 10. FIG. 3 is a cross-sectional view of the injector 200. As illustrated in FIG. 3, the injector assembly 10 is an assembly including an actuator 101, a container 102, an attachment 103, and a plunger 104.

The actuator 101 is a member that generates energy for the injector 200 to inject the injection objective substance. The actuator 101 includes a body 101a, the initiator 101b, and a piston 101c. The actuator 101 uses the initiator 101b as an activation source and the piston 101c as an output portion. The body 101a is formed in a tubular shape, and an opening 101d is formed at a distal end thereof. The initiator 101b is an electric igniter that releases a combustion product by combusting an ignition charge, and is fitted in the body 101a and thus closes a proximal end part of the body 101a. The piston 101c is disposed between the initiator 101b and the opening 101d and it can slide inside the body 101a in the long direction. A space between the initiator 101b and the piston 101c forms a combustion chamber 101e in which a combustion product is released.

Here, examples of the ignition charge used for the initiator 101b include an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and ferric oxide (AFO), and an explosive composed of a combination of a plurality of these explosives. These explosives exhibit characteristics that, although the explosives generate high-temperature and high-pressure plasma during combustion immediately after ignition, when the combustion product condenses at room temperature, the explosives do not contain gaseous components and hence the pressure generated decreases abruptly. As long as injection of an appropriate injection objective substance is possible, another explosive may be used as an ignition charge.

The container 102 is a member for injecting the injection objective substance. The container 102 is formed in a tubular shape, and is provided with a nozzle portion 10a having a circular cross section by reducing the diameter of the distal end thereof.

The attachment 103 is a member that couples the body 101a and the container 102. The attachment 103 is formed in a tubular shape, the actuator 101 is fitted into a proximal end side thereof, and the container 102 is fitted into a distal end side thereof. The inner circumferential surface of the attachment 103 and the outer circumferential surface of the body 101a are screwed together, and the inner circumferential surface of the attachment 103 and the outer circumferential surface of the container 102 are screwed together, whereby the body 101a and the container 102 are coupled with the attachment 103 interposed therebetween.

The plunger 104 is a member that pressurizes the injection objective substance by the energy received from the actuator 101. The plunger 104 is accommodated in the attachment 103 and is disposed between the actuator 101 and the nozzle portion 10a of the container 102. The plunger 104 is formed in a rod shape, and a proximal end part thereof is engaged with a distal end part of the piston 101c. A distal end part of the plunger 104 is inserted into the container 102, and a space between the plunger 104 and the nozzle portion 10a forms an accommodation portion 10c to be filled with the injection objective substance.

The housing 20 is a member that accommodates the injector assembly 10 and functions as a grip portion that a user grasps to use the injector 200. The outer surface of the housing 20 is provided with a plurality of switches (not illustrated) for operating the injector 200 to achieve injection of the injection objective substance. The plurality of switches are connected to a control unit (not illustrated) such as a microcomputer incorporated in the housing 20. The inner surface of the housing 20 is provided with a socket (not illustrated) connected to the initiator 101b of the injector assembly 10. Power is supplied to the control unit and the initiator 101b via the power cable 30 connected to the housing 20. The control unit controls supply of a drive current to the initiator 101b in response to a signal from each switch, thereby performing operation control of the injector 200.

When a drive current is supplied to the initiator 101b by a user's operation and the initiator 101b is activated, a combustion product is released from the initiator 101b to the combustion chamber 101e. When the pressure in the combustion chamber 101e increases, the piston 101c receives the pressure and slides toward the distal end side of the body 101a. Due to this, the plunger 104 engaged with the piston 101c is pushed toward the distal end side, and injection energy is applied to the injection objective substance accommodated in the accommodation portion 10c. Thus, the injection objective substance is injected from the injection port 10b formed at the distal end of the nozzle portion 10a. The kinetic energy of the injection objective substance ruptures the surface of the skin, and the injection objective substance is injected intradermally.

### [Aid 100]

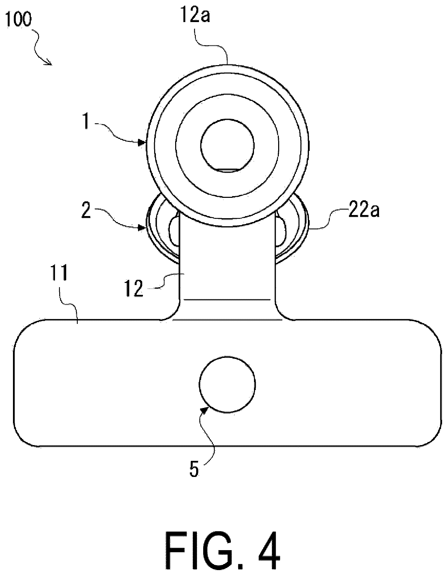

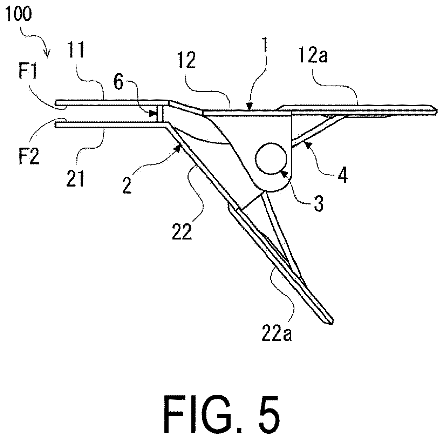

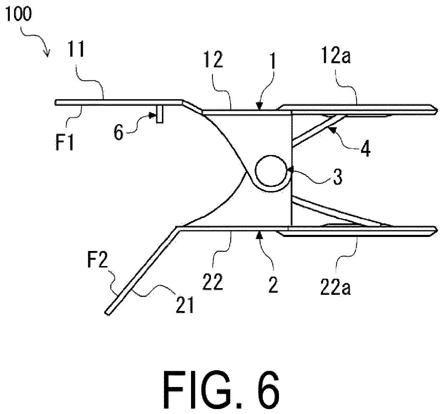

Next, the aid 100 according to the present embodiment will be described. FIG. 4 is a top view of the aid 100 in a closed state. FIG. 5 is a side view of the aid 100 in a closed state. FIG. 6 is a side view of the aid 100 in an open state. The aid 100 pinches the skin with a first pinching piece 11 and a second pinching piece 21 facing each other, thereby enabling intensive and reliable injection into the epidermis or the dermis with the injector 200. Hereinafter, details will be described with reference to the drawings.

The aid 100 is formed by processing what is called a bulldog clip. The state of the aid 100 can be changed between a closed state in which the first pinching piece 11 and the second pinching piece 21 are closed as illustrated in FIG. 5 and an open state in which the first pinching piece 11 and the second pinching piece 21 are opened as illustrated in FIG. 6. As illustrated in FIG. 5 and the like, the aid 100 includes a first pinching member 1, a second pinching member 2, a coupling member 3, and a biasing member 4.

The first pinching member 1 is a plate-like member formed in a T shape in top view and formed in a straight line shape in side view. The first pinching member 1 includes the first pinching piece 11 that is to abut on the skin and a first extension part 12 extending from one end of the first pinching piece 11. A first grasping part 12a that the user grasps to perform an operation of opening and closing the aid 100 is formed at a distal end of the first extension part 12. The second pinching member 2 is a plate-like member formed in a T shape in top view and formed in a V shape in side view. The second pinching member 2 includes the second pinching piece 21 that is to abut on the skin to pinch the skin in cooperation with the first pinching piece 11, and a second extension part 22 extending from one end of the second pinching piece 21. A second grasping part 22a that the user grasps to perform an operation of opening and closing the aid 100 is formed at a distal end of the second extension part 22. Note that the first grasping part 12a is not necessarily formed at the distal end of the first extension part 12, and is formed at at least a part of the first extension part 12. Similarly, the second grasping part 22a is formed at at least a part of the second extension part 22. Here, surfaces of the first pinching piece 11 and the second pinching piece 21 facing each other are referred to as pinching surfaces. As illustrated in FIGS. 5 and 6, the pinching surface of the first pinching piece 11 is referred to as a first pinching surface F1, and the pinching surface of the second pinching piece 21 is referred to as a second pinching surface F2. As illustrated in FIGS. 5 and 6, the first pinching surface F1 and the second pinching surface F2 are formed as flat surfaces. The shapes of the first pinching piece 11 and the second pinching piece 21 are not limited to the shapes illustrated in FIG. 4 and the like. The first pinching surface F1 and the second pinching surface F2 need not be flat surfaces, and may be formed as curved surfaces.

As illustrated in FIG. 4, a reception portion 5 that can receive the nozzle portion 10a of the injector 200 is formed in the first pinching piece 11. The reception portion 5 is a circular hole penetrating the first pinching piece 11 in a thickness direction thereof. The diameter of the reception portion 5 is equal to or slightly larger than the diameter of the nozzle portion 10a and thus the nozzle portion 10a and the reception portion 5 can be engaged with each other. The first pinching surface F1 of the first pinching piece 11 is provided with a positioning portion 6. The positioning portion 6 is formed as a protrusion protruding from the first pinching surface F1 of the first pinching piece 11 toward the second pinching surface F2 of the second pinching piece 21.

The coupling member 3 is a shaft member that pivotally couples the first pinching member 1 and the second pinching member 2. The coupling member 3 couples the first extension part 12 and the second extension part 22 such that the first pinching piece 11 and the second pinching piece 21 open and close by an operation of the first grasping part 12a and the second grasping part 22a. The coupling member 3 is an example of the "coupling portion" according to the present disclosure. The biasing member 4 is a spring member that biases the first pinching member 1 and the second pinching member 2 in a direction in which the aid 100 is brought into a closed state. The user grasps the first grasping part 12a and the second grasping part 22a of the aid 100 in a closed state and brings the first grasping part 12a and the second grasping part 22a close to each other against the biasing force of the biasing member 4, whereby the first pinching member 1 and the second pinching member 2 pivot on the coupling member 3 as an axis, and the aid 100 is changed from the closed state to the open state.

### [Injection Method]

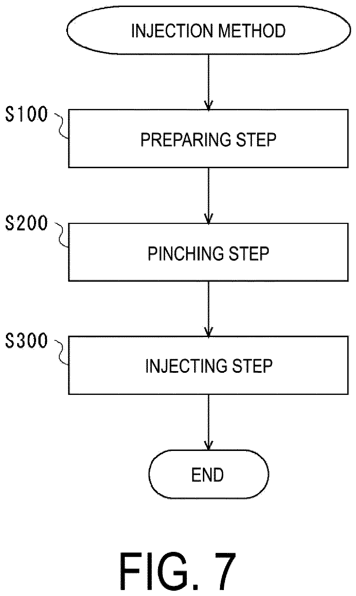

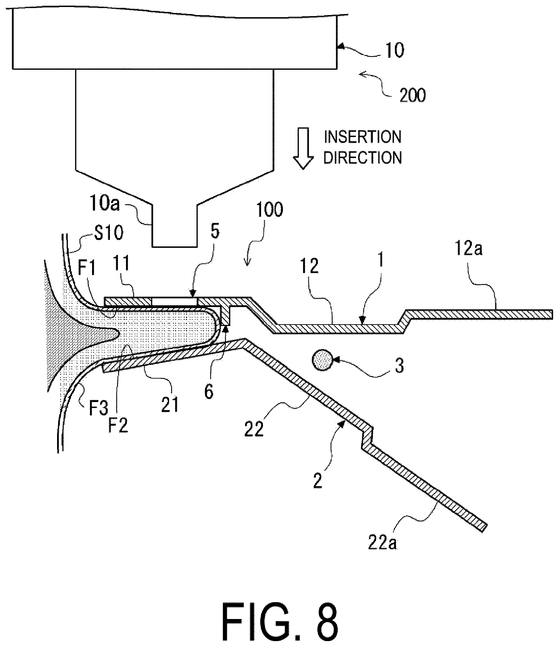

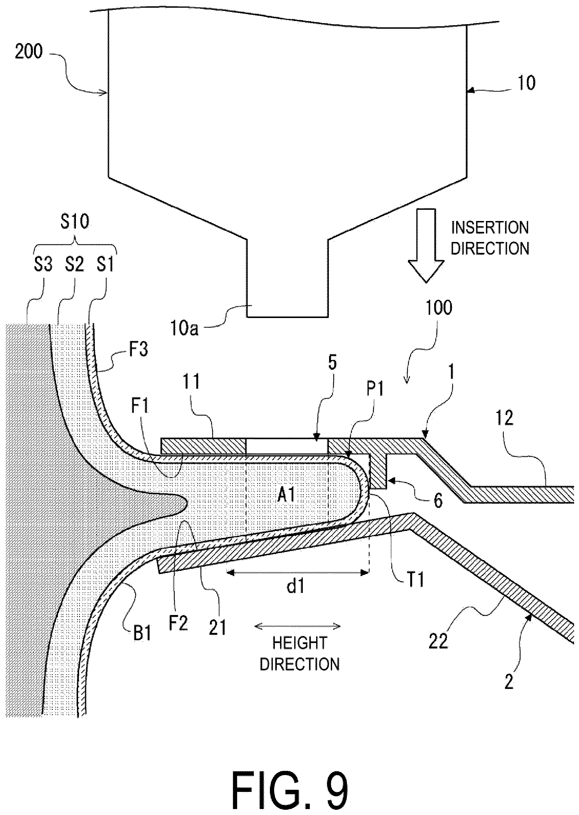

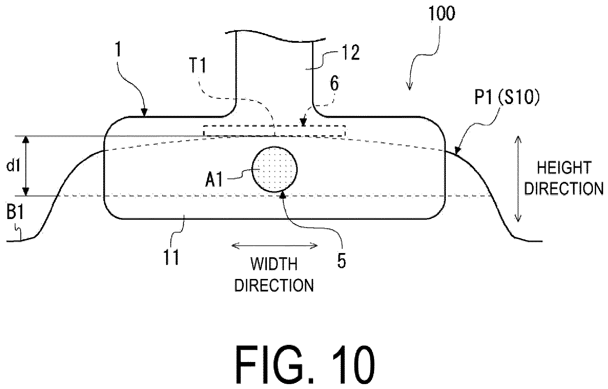

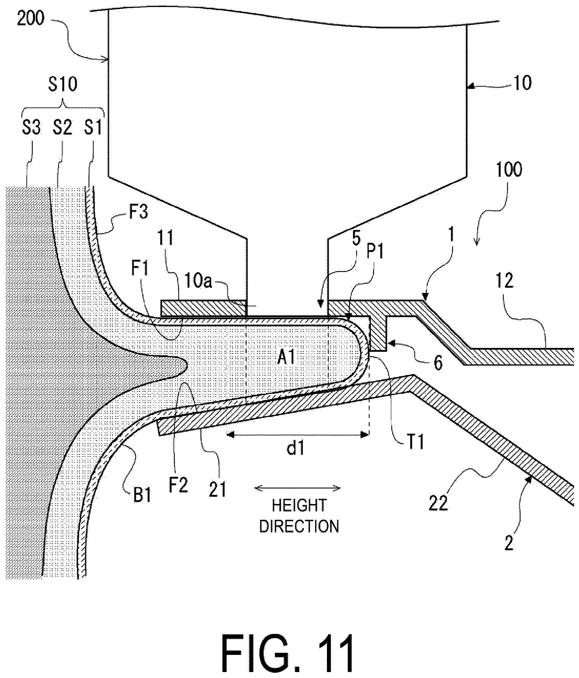

Next, a method for intradermal injection to a mammal with the injector 200 using the aid 100 according to the present embodiment will be described. FIG. 7 is a flowchart showing a procedure of the injection method using the aid 100. FIG. 8 is a cross-sectional view illustrating a state in which the skin is pinched by the aid 100. FIG. 9 is an enlarged view of FIG. 8. FIG. 10 is a top view illustrating a state in which the skin is pinched by the aid 100. FIG. 11 is a cross-sectional view illustrating a state in which the nozzle portion 10a of the injector 200 is received in the reception portion 5 of the aid 100. Reference sign S10 in FIG. 9 denotes the skin of a mammal. In FIG. 9, reference sign S1 denotes the epidermis, reference sign S2 denotes the dermis, and reference sign S3 denotes the subcutaneous tissue. The skin S10 includes three layers of the epidermis S1, the dermis S2, and the subcutaneous tissue S3. Reference sign F3 in FIG. 9 denotes the surface (hereinafter, skin surface) of the skin S10.

First, in a preparing step S100, the injector 200 and the aid 100 are prepared.

Next, in a pinching step S200, the skin S10 is pinched by the aid 100 as illustrated in FIG. 8. In the pinching step, the aid 100 is brought into an open state by a user's operation, and the skin S10 is tucked between the first pinching piece 11 and the second pinching piece 21, whereby the skin S10 is pinched. At this time, the state in which the skin S10 is pinched between the first pinching piece 11 and the second pinching piece 21 is maintained by the biasing force of the biasing member 4 to bring the aid 100 into a closed state.

Here, as illustrated in FIG. 9, the first pinching piece 11 and the second pinching piece 21 pinch the skin S10 in a state of pinching the skin S10 with the first pinching surface F1 and the second pinching surface F2 facing each other, to form a fold shape part P1 in which the skin S10 is raised in a fold shape. In the fold shape part P1, the epidermis S1 is deformed and thus bent in a U shape. Reference sign T1 in FIG. 9 denotes a most protruding site in the fold shape part P1, that is, a top part (hereinafter, fold top part) of the fold shape part P1. The fold top part T1 is a bent part of the epidermis S1. Reference sign B1 in FIG. 9 denotes a root part (hereinafter, fold root part) of the fold shape part P1. The fold root part B1 is a proximal end part of the fold shape part P1. As illustrated in FIG. 9, a direction in which the fold shape part P1 protrudes is defined as a height direction of the fold shape part P1.

At this time, as illustrated in FIGS. 9 and 10, the skin S10 is pinched such that the fold top part T1 abuts on the positioning portion 6, whereby the fold top part T1 is positioned. Consequently, the skin surface F3 in a fold top part vicinity region A1, which is a region in the vicinity of the fold top part T1, in the fold shape part P1 is exposed through the reception portion 5. Specifically, in the fold shape part P1, the fold top part vicinity region A1 is a region positioned in a range within a distance d1 from the fold top part T 1 to the fold root part B1 side in the height direction of the fold shape part P1.

Next, in an injecting step S300, an injection objective substance is injected into the fold top part vicinity region A1. In the injecting step, first, as illustrated in FIG. 10, the nozzle portion 10a of the injector 200 is received (inserted) into the reception portion 5 of the aid 100. At this time, the nozzle portion 10a is inserted into the reception portion 5 along a direction orthogonal to the first pinching piece 11, that is, along a direction perpendicular to the skin surface F3 in the fold top part vicinity region A1. Here, as illustrated in FIG. 8, the first extension part 12 of the first pinching member 1 of the aid 100 extends from the first pinching piece 11 and the first pinching piece 11 and the first extension part 12 form a straight line shape, and the second extension part 22 of the second pinching member 2 extends from the second pinching piece 21 and the second pinching piece 21 and the second extension part 22 form a V shape. That is, the first extension part 12 extends so as not to be positioned on the front side relative to the first pinching piece 11 in the insertion direction of the nozzle portion 10a with respect to the reception portion 5. Therefore, when the nozzle portion 10a is received in the reception portion 5, the nozzle portion 10a does not interfere with the first extension part 12.

When the nozzle portion 10a is received in the reception portion 5, the reception portion 5 is engaged with the nozzle portion 10a, and the injection port 10b is positioned with respect to the fold top part vicinity region A1 where the skin surface F3 is exposed through the reception portion 5. As illustrated in FIG. 9, the nozzle portion 10a is perpendicularly pressed against the skin surface F3 in the fold top part vicinity region A1 of the fold shape part P1, and the injection port 10b is brought into a state of facing the fold top part vicinity region A1. At this time, the nozzle portion 10a is pressed against the fold top part vicinity region A1, and the injection port 10b is brought into a state of being in contact with the skin surface F3 in the fold top part vicinity region A1. In this state, the user operates the injector 200 and injects the injection objective substance from the injection port 10b to the fold top part vicinity region A1, whereby the skin surface F3 in the fold top part vicinity region A1 ruptures, and the injection objective substance is injected intradermally to the fold top part vicinity region A1. Here, as illustrated in FIG. 9, since the fold shape part P1 is formed in a state in which the skin S10 is pinched, the fold top part vicinity region A1 includes the epidermis S1 and the dermis S2 and does not include the subcutaneous tissue S3. Therefore, the injection objective substance is intensively and reliably injected into the epidermis S1 or the dermis S2.

Furthermore, since the second pinching surface F2 of the second pinching piece 21 positioned on the opposite side of the nozzle portion 10a with the fold shape part P1 interposed therebetween is formed as a flat surface, the fold shape part P1 and the second pinching surface F2 are in air-tightly contact with each other. Therefore, the fold shape part P1 is stably supported by the second pinching surface F2, and the injection objective substance is reliably injected to the fold top part vicinity region A1.

### [Actions and Effects]

As described above, in the aid 100 according to the present embodiment, the first pinching piece 11 and the second pinching piece 21 facing each other pinch the skin S10 in a state of pinching the skin S10 with the first pinching surface F1 and the second pinching surface F2 facing each other, to form the fold shape part P1 in which the skin S10 is raised in a fold shape. Then, the reception portion 5 provided in the first pinching piece 11 exposes the fold top part vicinity region A1, which is a region in the vicinity of the fold top part T1, in the fold shape part P 1, and can receive the nozzle portion 10a of the injector 200.

Thus, the injection objective substance is injected from the injection port 10b formed in the nozzle portion 10a in a state where the nozzle portion 10a of the injector 200 is received in the reception portion 5, whereby the injection objective substance is injected to the fold top part vicinity region A1. As a result, the aid 100 according to the present embodiment allows intensive and reliable injection of the injection objective substance into the epidermis S1 or the dermis S2 in intradermal injection to a mammal with the jet injector.

Here, it is known that delivering a specific injection objective substance such as a vaccine or a gene therapy agent to the epidermis or the dermis allows a response to the injection objective substance to be effectively achieved as compared with the case of delivering the injection objective substance to the subcutaneous tissue. Therefore, also for a jet injector, it may be preferable to intensively and reliably inject an injection objective substance into the epidermis or the dermis. In an injection method using a known jet injector, injection into the epidermis or the dermis is performed by adjusting the output of the jet injector for injecting an injection objective substance. However, since the thickness of the skin varies depending on the type of the animal, site, sex, age (age in weeks), and the like, it has been difficult to intensively and reliably inject an injection objective substance into the epidermis or the dermis.

On the other hand, since the aid 100 according to the present embodiment allows intensive and reliable injection of an injection objective substance into the epidermis S1 or the dermis S2, effective expression of an intradermally administered gene can be achieved. As a result, it is possible to effectively achieve a response to a specific injection objective substance such as a vaccine or a gene therapy agent.

Here, the reception portion 5 may expose a region positioned in a range within 3 mm from the fold top part T1 to the fold root part B1 side in the fold shape part P1. That is, assuming d1 = 3 mm, a region positioned in a range within 3 mm from the fold top part T1 to the fold root part B1 side in the fold shape part P1 may be exposed as the fold top part vicinity region A1 through the reception portion 5. When the injection objective substance is injected into such a fold top part vicinity region A1, the injection objective substance is injected at a position within 3 mm from the fold top part T1 to the fold root part B1 side. As a result, it is possible to intensively and reliably inject the injection objective substance into the epidermis S1 or the dermis S2.

The aid 100 according to the present embodiment includes the positioning portion 6 that is provided on the first pinching surface F1 of the first pinching piece 11 and positions the fold top part T1 by abutting on the fold top part T1 of the fold shape part P1. Thus, the fold top part vicinity region A1 can be reliably exposed through the reception portion 5. Note that the positioning portion is not an essential component in the technique according to the present disclosure. When the aid according to the present disclosure includes the positioning portion, the positioning portion is provided on the pinching surface of at least one of the first pinching piece or the second pinching piece. For example, the positioning portion may be provided only on the pinching surface of the second pinching piece, or the positioning portion may be provided on the pinching surfaces of both of the first pinching piece and the second pinching piece.

The reception portion 5 of the aid 100 according to the present embodiment is configured such that it positions the injection port 10b with respect to the fold top part vicinity region A1 by engaging with the nozzle portion 10a of the injector 200. This suppresses displacement of the nozzle portion 10a from the fold top part vicinity region A1 during injection, and the injection objective substance can be more reliably injected to the fold top part vicinity region A1.

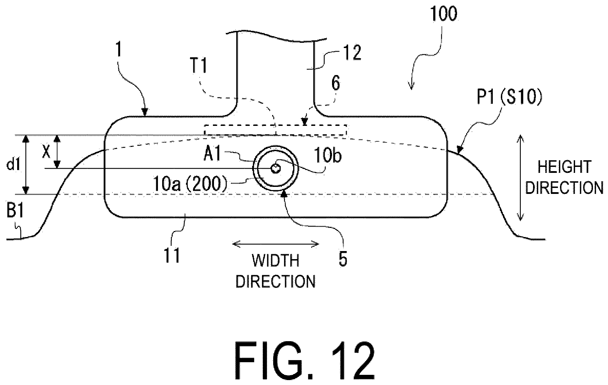

FIG. 12 is a top view illustrating a state in which the nozzle portion of the jet injector is received in the reception portion of the aid according to the first embodiment. Reference sign X in FIG. 12 denotes a distance between the injection port 10b of the nozzle portion 10a engaged with the reception portion 5 and the fold top part T1 in the height direction of the fold shape part P1. At this time, X ≤ 3 mm may be satisfied. That is, by engaging with the nozzle portion 10a of the injector 200, the reception portion 5 may position the injection port 10b such that the injection port 10b is positioned within 3 mm from the fold top part T1 to the fold root part B1 side in the fold shape part P1. This enables the injection objective substance to be reliably injected at a position within 3 mm from the fold top part T1 to the fold root part B1 side. As a result, it is possible to intensively and reliably inject the injection objective substance into the epidermis S1 or the dermis S2.

Furthermore, X ≤ 1.5 mm may be satisfied. That is, by engaging with the nozzle portion 10a, the reception portion 5 may position the injection port 10b such that the injection port 10b is positioned within 1.5 mm from the fold top part to the fold root part side in the fold shape part. This enables the injection objective substance to be reliably injected at a position within 1.5 mm from the fold top part T1 to the fold root part B1 side. As a result, it is possible to more intensively and reliably inject the injection objective substance into the epidermis S1 or the dermis S2.

The second pinching surface F2 of the second pinching piece 21 of the aid 100 according to the present embodiment is formed as a flat surface. Thus, the fold shape part P1 and the second pinching surface F2 are brought into air-tightly contact with each other, the fold shape part P1 is stably supported by the second pinching surface F2, and the injection objective substance can be reliably injected to the fold top part vicinity region A1.

The first extension part 12 of the aid 100 according to the present embodiment extends such that the nozzle portion 10a of the injector 200 does not interfere with the first extension part 12 when the nozzle portion 10a is received in the reception portion 5. This allows the nozzle portion 10a to be smoothly received in the reception portion 5.

### [Administration Experiment]

An experiment was conducted to confirm the effect of the method for intradermal injection to a mammal with a jet injector using the aid according to the embodiment. In the experiment, as an injection objective substance, 30 µL (1 µg/µL) of a luciferase expression plasmid (pGL3 manufactured by Promega Corporation) was administered (injected) into the flank of an SD rat (♀8 weeks) using the jet injector. In 24 hours after administration, the administration site was excised using a 5 mm diameter punch, accommodated in a 2 mL microtube, and stored in a freezer at -80°C.

The luciferase activity was measured using a luciferase assay kit (Catalog No. E1500) manufactured by Promega Corporation. The frozen skin was thawed in 500 µL of a tissue lysate and cut to approximately 1.5 mm or less using scissors. Thereafter, the mixture was freeze-dissolved three times. To 20 µL of the supernatant obtained by centrifuging the lysate, 100 µL of a substrate solution was added, and the mixture was stirred, and then a relative light unit (RLU) was measured using a P-100 device manufactured by Kikkoman Corporation, whereby the expression level of a luciferase gene was measured.

### [Examples]

In Examples 1 to 4, the injection was performed by the injection method using the aid according to the first embodiment. That is, the skin that was an administration target was pinched using the aid according to the first embodiment, and the luciferase expression plasmid was injected to the fold top part vicinity region with the jet injector. In Example 1, the distance X from the fold top part (folded part of the skin) to the injection port of the nozzle portion in the height direction of the fold shape part was set to X = 1 mm. The distance X was set to 1 mm < X < 2 mm in Example 2, X = 2 mm in Example 3, and X = 3 mm in Example 4.

### [Comparative Example]

In Comparative Example, administration was performed with a known jet injector. That is, without using the aid according to the present embodiment, the nozzle portion of the jet injector was perpendicularly pressed against the skin that was an administration target, and the luciferase expression plasmid was injected.

### [Experiment Results]

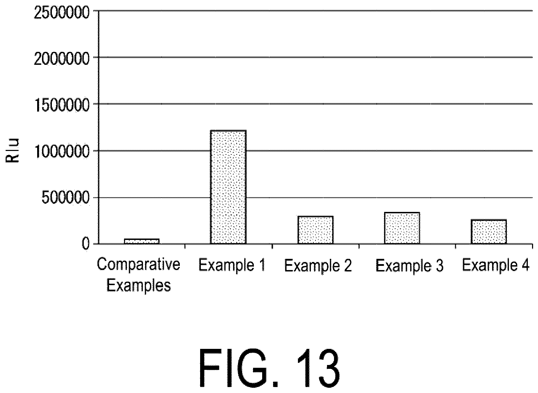

FIG. 13 is a graph showing the relative light units of Examples 1 to 4 and Comparative Example. Comparison between Examples 1 to 4 with Comparative Example indicates that the expression levels of genes in all of Examples 1 to 4 are higher than that in Comparative Example. Thus, the effect of use of the aid according to the embodiment, that is, the fact that the injection objective substance can be intensively injected into the epidermis or the dermis of the skin was confirmed. Comparison among Examples 1 to 4 indicates that Example 1, in which X = 1 mm, has the highest gene expression level. This suggests that the above effect can be further enhanced by injecting the injection objective substance at a position closer to the fold top part (folded part of the skin).

### [Modified Example]

Hereinafter, an aid according to a modified example of the first embodiment will be described. In the description of the modified example, a configuration different from that of the aid 100 described in FIG. 1 and the like will be mainly described, and similar configurations to those of the aid 100 are given the same reference signs as those of the aid 100, and thus detailed description will be omitted.

### [First Modified Example]

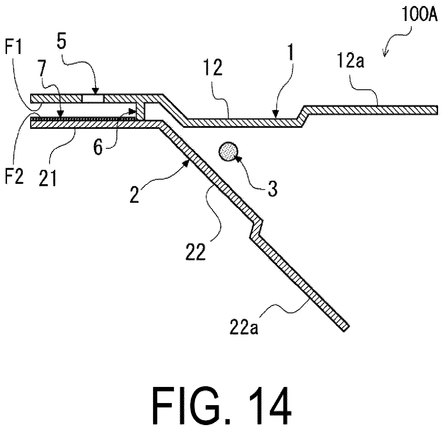

FIG. 14 is a cross-sectional view of an aid 100A according to the first modified example of the first embodiment. As illustrated in FIG. 14, the aid 100A is different from the aid 100 in that the second pinching surface F2 of the second pinching piece 21 is provided with a non-slip portion 7. The non-slip portion 7 is for suppressing slippage of the fold shape part P1 pinched by the first pinching piece 11 and the second pinching piece 21. A material of the non-slip portion 7 is not particularly limited, but for example, the non-slip portion 7 can be formed by coating the second pinching surface F2 with a silicon resin.

The aid 100A according to the first modified example of the first embodiment allows suppression of positional displacement of the fold shape part P1 with respect to the aid 100 during injection. This can maintain a state in which the fold top part vicinity region A1 is exposed through the reception portion 5, and allows more reliable injection of the injection objective substance to the fold top part vicinity region A1. When the aid according to the present disclosure includes the non-slip portion, the non-slip portion is provided on the pinching surface of at least one of the first pinching piece or the second pinching piece. For example, the non-slip portion may be provided only on the pinching surface of the first pinching piece, or the non-slip portion may be provided on the pinching surfaces of both of the first pinching piece and the second pinching piece.

### [Second Modified Example]

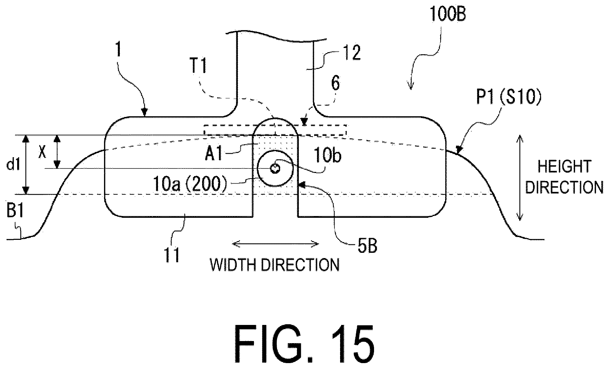

FIG. 15 is a top view illustrating a state in which the nozzle portion 10a of the injector 200 is received in a reception portion 5B of an aid 100B according to the second modified example of the first embodiment. As illustrated in FIG. 15, the aid 100B is different from the aid 100 in that the reception portion 5B is formed in a slit shape. The reception portion 5B according to the second modified example is formed as a slit provided in the first pinching piece 11 and extends along the height direction of the fold shape part P1. When the nozzle portion 10a of the injector 200 is received in the reception portion 5B, the nozzle portion 10a and the reception portion 5B are engaged with each other. In a state where the nozzle portion 10a and the reception portion 5B are engaged with each other, the movement of the nozzle portion 10a in a width direction of the fold shape part P1 is restricted, and the movement of the nozzle portion 10a in a height direction of the fold shape part P1 is allowed. Therefore, by sliding the nozzle portion 10a along the extending direction of the reception portion 5B (that is, the height direction of the fold shape part P1) in a state where the nozzle portion 10a and the reception portion 5B are engaged with each other, it is possible to change the position (that is, the distance X) of the injection port 10b in the height direction of the fold shape part P 1.

### [Third Modified Example]

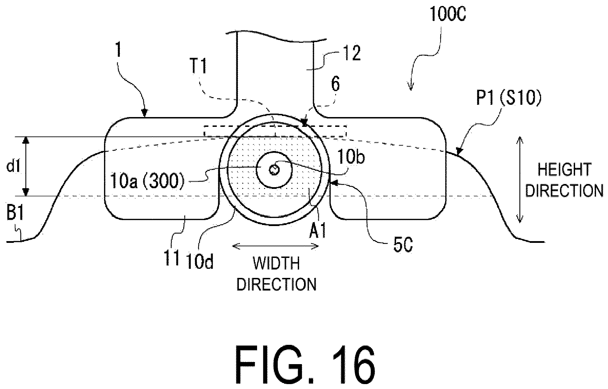

FIG. 16 is a top view illustrating a state in which the nozzle portion 10a of an injector 300 according to a modified example is received in a reception portion 5C of an aid 100C according to the third modified example of the first embodiment. As illustrated in FIG. 16, the aid 100C is different from the aid 100 in that a pressing portion 10d is formed such that it is receivable in the reception portion 5C. As disclosed in JP 2020-31715 A, the pressing portion 10d receivable in the reception portion 5C according to the third modified example is provided surrounding the nozzle portion 10a and forming a predetermined gap between the pressing portion 10d and the nozzle portion 10a. This makes it easy to form and maintain a suitable contact state between the skin S10, which is an injection target region, and the nozzle portion 10a.

### <Second Embodiment>

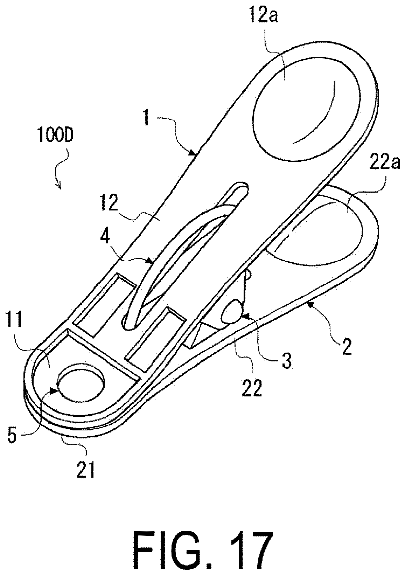

FIG. 17 is an overall view of an aid 100D according to the second embodiment. In FIG. 17, similar configurations to those of the aid 100 according to the first embodiment are given the same reference signs as those of the aid 100. An aid 100D according to the second embodiment is formed by processing what is called a clothespin. The aid 100D according to the second embodiment includes the first pinching piece 11, the second pinching piece 21, and the reception portion 5, similarly to the aid 100 according to the first embodiment. Therefore, also with the aid 100D, as in the first embodiment, it is possible to intensively and reliably inject the injection objective substance into the epidermis S1 or the dermis S2 in intradermal injection to a mammal with a jet injector.

### <Third Embodiment>

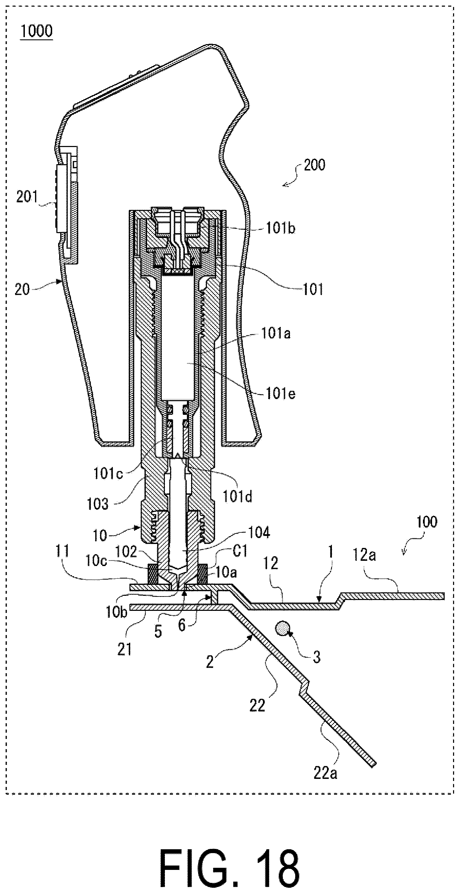

FIG. 18 is a cross-sectional view of a jet injector 1000 with an aid according to the third embodiment. In the jet injector 1000 with an aid, the injector 200 and the aid 100 according to the first embodiment are coupled via a coupling means C1. As illustrated in FIG. 18, in the jet injector 1000 with an aid, the injector 200 and the aid 100 are coupled in a state where the nozzle portion 10a is received in the reception portion 5.

When performing intradermal injection to a mammal using the jet injector 1000 with an aid, the injector 200 is operated in a state where the skin S10 is pinched by the aid 100 and the fold shape part P1 is formed, and an injection objective substance is injected from the injection port 10b to the fold top part vicinity region A1 exposed through the reception portion 5. In the jet injector 1000 with an aid, the nozzle portion 10a is received in the reception portion 5 and the injection port 10b faces the fold top part vicinity region A1 of the fold shape part P1. Therefore, it is possible to omit the operation of inserting the nozzle portion 10a into the reception portion 5 after the skin S10 is pinched by the aid 100.

Note that the coupling means C1 is not an essential component in the technique according to the present disclosure, and it is sufficient that the aid and the jet injector are coupled in a state where the nozzle portion is received in the reception portion.

### <Others>

Suitable embodiments according to the present disclosure have been described above, but each embodiment disclosed in the present specification can be combined with each of features disclosed in the present specification.

Regarding the above-described embodiments and modified examples, the following supplementary notes are further disclosed.

### <Supplementary Note 1>

An injection method for intradermally injecting an injection objective substance to a mammal by injecting the injection objective substance from an injection port formed in a nozzle portion of a jet injector, the injection method including:
preparing the jet injector, and an aid including a first pinching piece and a second pinching piece facing each other and a reception portion that is provided in the first pinching piece and can receive the nozzle portion;
pinching a skin in a state of holding the skin with pinching surfaces facing each other of the first pinching piece and the second pinching piece and thus forming a fold shape part in which the skin is raised in a fold shape, and exposing, through the reception portion, a fold top part vicinity region, which is a region in a vicinity of a fold top part, in the fold shape part; and
receiving the nozzle portion of the jet injector in the reception portion in a state where the skin is pinched, and injecting the injection objective substance from the injection port to the fold top part vicinity region.

### <Supplementary Note 2>

The injection method according to Supplementary Note 1, in which in injecting the injection objective substance from the injection port, the injection objective substance is injected at a position within 3 mm from the fold top part to a fold root part side in the fold shape part.

### <Supplementary Note 3>

The injection method according to Supplementary Note 1 or 2, in which in injecting the injection objective substance from the injection port, the injection objective substance is injected at a position within 1.5 mm from the fold top part to a fold root part side in the fold shape part.

### <Supplementary Note 4>

The injection method according to any one of Supplementary Notes 1 to 3, in which the injection objective substance contains a gene.

### Reference Signs List

1: First pinching member
2: Second pinching member
3: Coupling member
4: Biasing member
5: Reception portion
6: Positioning portion
7: Non-slip portion
10a: Nozzle portion
10b: Injection port
11: First pinching piece
12: First extension part
12a: First grasping part
21: Second pinching piece
22: Second extension part
22a: Second grasping part
100: Aid
200: Jet injector
1000: Jet injector with aid

## Claims

1. An aid to be applied to a jet injector that intradermally injects an injection objective substance by injecting the injection objective substance from an injection port formed in a nozzle portion, the aid comprising:
a first pinching piece and a second pinching piece that face each other, and pinch a skin in a state of holding the skin with pinching surfaces facing each other and thus form a fold shape part where the skin is raised in a fold shape; and
a reception portion that is provided in the first pinching piece, exposes a fold top part vicinity region, which is a region in a vicinity of a fold top part, in the fold shape part, and can receive the nozzle portion.

2. The aid according to claim 1, wherein the fold top part vicinity region is a region positioned in a range within 3 mm from the fold top part to a fold root part side in the fold shape part.

3. The aid according to claim 1 or 2 further comprising:
a positioning portion that is provided on the pinching surface of at least one of the first pinching piece or the second pinching piece and positions the fold top part by abutting on the fold top part of the fold shape part.

4. The aid according to any one of claims 1 to 3, wherein the reception portion positions the injection port with respect to the fold top part vicinity region by being engaged with the nozzle portion of the jet injector.

5. The aid according to claim 4, wherein the reception portion positions the injection port by being engaged with the nozzle portion of the jet injector and the injection port is positioned within 3 mm from the fold top part to the fold root part side in the fold shape part.

6. The aid according to claim 4, wherein the reception portion positions the injection port by being engaged with the nozzle portion of the jet injector and the injection port is positioned within 1.5 mm from the fold top part to the fold root part side in the fold shape part.

7. The aid according to any one of claims 1 to 6, wherein the pinching surface of the second pinching piece is formed as a flat surface.

8. The aid according to any one of claims 1 to 7, wherein the pinching surface of at least one of the first pinching piece or the second pinching piece is provided with a non-slip portion for suppressing slippage of the fold shape part.

9. The aid according to any one of claims 1 to 8, comprising:
a first extension part extending from one end of the first pinching piece and provided with a first grasping part at least in a part of the first extension part, a second extension part extending from one end of the second pinching piece and provided with a second grasping part at least in a part of the second extension part, and a coupling portion coupling the first extension part and the second extension part such that the first pinching piece and the second pinching piece open and close by an operation of the first grasping part and the second grasping part.

10. The aid according to claim 9, wherein the first extension part extends such that the nozzle portion does not interfere with the first extension part when the nozzle portion is received in the reception portion.

11. The aid according to claim 10, wherein
the first extension part extends from the first pinching piece and the first pinching piece and the first extension part form a straight line shape, and
the second extension part extends from the second pinching piece and the second pinching piece and the second extension part form a V shape.

12. A jet injector with an aid, comprising:
a jet injector that intradermally injects an injection objective substance by injecting the injection objective substance from an injection port formed in a nozzle portion, and an aid coupled to the jet injector, wherein
the aid includes:
a first pinching piece and a second pinching piece that face each other, and pinch a skin in a state of holding the skin with pinching surfaces facing each other and thus form a fold shape part where the skin is raised in a fold shape, and
a reception portion that is provided in the first pinching piece and receives the nozzle portion such that the injection port faces a fold top part vicinity region, which is a region in a vicinity of a fold top part, in the fold shape part.

13. An injection method for intradermally injecting an injection objective substance to a mammal (excluding a human) by injecting the injection objective substance from an injection port formed in a nozzle portion of a jet injector, the injection method comprising:
preparing the jet injector, and an aid including a first pinching piece and a second pinching piece facing each other and a reception portion that is provided in the first pinching piece and can receive the nozzle portion;
pinching a skin in a state of holding the skin with pinching surfaces facing each other of the first pinching piece and the second pinching piece and thus forming a fold shape part in which the skin is raised in a fold shape, and exposing, through the reception portion, a fold top part vicinity region, which is a region in a vicinity of a fold top part, in the fold shape part; and
receiving the nozzle portion of the jet injector in the reception portion in a state where the skin is pinched, and injecting the injection objective substance from the injection port to the fold top part vicinity region.

14. The injection method according to claim 13, wherein in injecting the injection objective substance from the injection port, the injection objective substance is injected at a position within 3 mm from the fold top part to a fold root part side in the fold shape part.

15. The injection method according to claim 13, wherein in injecting the injection objective substance from the injection port, the injection objective substance is injected at a position within 1.5 mm from the fold top part to a fold root part side in the fold shape part.

16. The injection method according to any one of claims 13 to 15, wherein the injection objective substance contains a gene.
